# EUROPEAN PATENT SPECIFICATION

(11) **EP 4 382 172 B1**
(45) Date of publication and mention of the grant of the patent: **11.03.2026**
(21) Application number: 22212050.3
(22) Date of filing: 07.12.2022
(51) Int. Cl.: A61Q 5/04, A61K 8/46, A61K 8/49

(54) **COMPOSITION FOR PERMANENT SHAPING OF HUMAN HAIR**
ZUSAMMENSETZUNG ZUR DAUERHAFTEN VERFORMUNG VON MENSCHLICHEN HAAREN
COMPOSITION POUR LA MISE EN FORME PERMANENTE DES CHEVEUX HUMAINS

(43) Date of publication of application: 12.06.2024
(73) Proprietor: Kao Germany GmbH, 64297 Darmstadt (DE)
(72) Inventor: ILHAM, Stephanie, 64297 Darmstadt (DE); BAUER, Peter, 64297 Darmstadt (DE); BREAKSPEAR, Steven, 64297 DARMSTADT (DE); NÖCKER, Bernd, 64297 DARMSTADT (DE)

(56) References cited:
- EP-A2- 2 347 752
- US-A1- 2009 041 700
- US-A1- 2013 276 809
- DATABASE GNPD [online] MINTEL; 7 September 2020 (2020-09-07), ANONYMOUS: "Treatment", XP093044726, retrieved from https://www.gnpd.com/sinatra/recordpage/8084277/ Database accession no. 8084277
- DATABASE GNPD [online] MINTEL; 7 September 2020 (2020-09-07), ANONYMOUS: "Hair Mask", XP093044729, retrieved from https://www.gnpd.com/sinatra/recordpage/8084279/ Database accession no. 8084279

## Description

The present invention relates to a composition for the permanent shaping of human hair, used both for the permanent waving of human hair with satisfactory waving effect as well as for the straightening of either naturally or chemically curled hair, comprising one or more reducing agents and ectoin and/or hydroxyectoin.

Consumers are continuously willing to change their hairstyle permanently by changing their straight hair to a more wavy or curly appearance by using curling processes or by changing to straight hair to eliminate frizz and unwanted volume by using straightening processes. In both cases, the hair is damaged, which may have bigger consequences for further processing of the hair.

It is generally known that permanent waving and straightening of hair is carried out in two steps, firstly the reductive splitting of the cysteine disulfide bonds in the hair by a reducing agent, and then the subsequent neutralization by application of an oxidizing agent, whereby the cysteine disulfide bonds are restored.

The reducing agent most frequently been used is thioglycolic acid, also in form of the salts thereof, especially its ammonium salt, although numerous other thio compounds have been proposed for this purpose, which, however, mostly did not succeed because of mainly toxicological reasons.

The compositions containing thioglycolates are customarily applied at a pH-value in the range of 7 and 10, in particular between 7.5 and 9.5.

Such compositions vary in their waving and/or straightening performance and, therefore, there is still need for further improvement. One of the major problems is varying efficiency of conventional permanent shaping compositions on hair having various levels of damage in its lengths. In other words, hair towards the roots is mostly healthy, and towards the tips is mostly damaged. The problem is aggravated on chemically processed hair. The conventional compositions deliver good permanent shaping performance on healthy hair whereas the efficiency is relatively poor on damaged hair or vice versa. There is, therefore, need for permanent shaping compositions having efficient permanent shaping effect independent from the damage level.

Additionally, strong alkaline pH together with the application of heat may elevate damaging effects on hair and prevent, on one hand a cosmetically attractive beautiful hair appearance and, on the other hand, may have negative effects on subsequent hair treatments. Therefore, there is a clear need for further developments for preventing the damage which may, furthermore, improve other hair properties and permanent shaping effects.

The inventors of the present invention have unexpectedly found that some damaging effects of the permanent shaping compositions and processes may be overcome by adding ectoin and/or hydroxyectoin into the composition, which additionally extends the lifetime of permanent waves and, also, hair straightened with such compositions remains in its straight shape for a longer period of time.

EP1513484 discloses ectoin to be used in perming processes in between reducing and oxidizing steps as an additional treatment step. The document is silent on compositions comprising ectoin and reducing agents.

WO202061659 discloses the reduction in hair damage with stable hair treatment compositions comprising thiol-based reducing agents and having pH values below 7.0, wherein most of the well-known reducing agents do not have their optimal effect. WO2009060014 discloses agents for dyeing and/or permanently shaping hair comprising bioflavonoids. Quantitative compositions relate solely hair dyeing compositions and shaping compositions have not been disclosed and Ectoin and its derivatives have only been mentioned as a possible compound for reducing skin irritation.

EP2347752 discloses cosmetic agents for changing the colour and/or shape of hair and having improved skin compatibility, in particular to reduce the potential for skin irritation and thus to minimize the potential for allergies. Ectoin is cited as a possible care substance.

US2013/276809 and US2009/041700 disclose compositions and processes for the permanent shaping of human hair used for the permanent waving of human hair with an excellent waving effect as well as straightening effect of curled hair. These documents are silent on compositions comprising ectoin or hydroxyectoin.

It is the understanding of the inventors that there is no teaching in prior art on ectoin and hydroxyectoin content of permanent shaping compositions and on their damage reducing properties and also extending the permanent shaping effects so that the shape change may be kept for a longer period.

Accordingly, the first object of the present invention is an aqueous composition for permanent shaping human hair comprising one or more reducing agents at a total concentration in the range of 2 to 20% by weight, calculated to the total of the composition, and ectoin and/or hydroxyectoin and having a pH in the range of 7.5 to 10.5.

The second object of the present invention is a method of permanent waving human hair wherein,
- the hair is washed and/or shampooed and towel dried,
- a composition of the present invention is applied onto hair and left on the hair for 1 to 45 min, preferably 1 to 30 min,
- the hair is rinsed off
- the hair is applied an oxidizing composition comprising at least one oxidizing agent, preferably hydrogen peroxide or sodium bromate at a concentration of 0.5 to 10% by weight, calculated to total of oxidizing composition, and left on the hair 1 to 20 min,
- the hair is rinsed off and dried,
wherein the hair is put on curlers before or during or after application of the composition according to claims 1 to 10 and the curlers are removed from hair during processing the oxidizing composition or after rinsing off the oxidizing composition.

The third object of the present invention is a method for straightening human hair wherein
- the hair is washed and/or shampooed and towel dried,
- a composition of the present invention is applied onto hair and left on the hair for 1 to 45 min, preferably 1 to 30 min,
- the hair is rinsed off,
- the hair is dried with a hair drier,
- the dry hair is physically straightened with a hot iron having a surface temperature in the range of 130 to 210°C,
- the hair is applied an oxidizing composition comprising at least one oxidizing agent, preferably hydrogen peroxide or sodium bromate at a concentration of 0.5 to 10% by weight calculated to total of oxidizing composition, and left on the hair 1 to 20 min, and
- the hair is rinsed off and dried.

The fourth object of the present invention is a kit for permanent shaping human hair comprising a first product comprising a composition of the present invention, a second product comprising a composition comprising at least one oxidizing agent and optionally a third product comprising a composition comprising at least one ectoin and/or hydroxyectoin.

The composition of the present invention comprises one or more reducing agents.

Suitable are thioglycolic acid, thiolactic acid and their salts, cystein and its hydrochloride salt, acetylcystein, glycerylthioglycolate and thioglycolic acid esters. The preferred are thioglycolic acid and its salts, cystein and its hydrochloride salt, and acetylcysteine. The most preferred is thioglycolic acid and its salts, especially its ammonium salt.

The composition comprises one or more reducing agents at a total concentration in the range of 2 to 20%, preferably 2.5 to 17%, more preferably 3 to 15%, and most preferably 5 to 12.5% by weight, calculated to the total of the composition.

The pH of the composition is in the range of 7.5 to 10.5, preferably 8 to 10.5, more preferably 8 to 10, and most preferably 8.5 to 10.

The composition comprises ectoin and/or hydroxyectoin at a concentration in the range of 0.001 to 15%, preferably 0.01 to 12.5%, more preferably 0.02 to 10%, and most preferably 0.1 to 5% by weight, calculated to the total of the composition.

The composition comprises one or more alkalizing agent selected from ammonia and/or its salts, ammonium carbamate, ammonium (bi)carbonate, monoethanolamine, triethanolamine, and tris(hydroxymethyl)aminomethane (TRIS).

The concentration of one or more alkalizing agents is in the range of 1 to 35%, preferably 2 to 30%, more preferably 2.5 to 25%, and most preferably 3 to 20% by weight, calculated to the total of the composition. The concentration of the agents may be dependent upon the type of the alkalizing agent used to adjust the pH and alkalinity of the composition.

Aqueous composition may comprise a thickening agent, preferably a thickening polymer. Suitable and preferred ones are thickening polymers such as polysaccharides such as alginate, pectinate, xanthan, hydroxypropyl xanthan or dehydroxanthan, non-ionic polysaccharides such as cellulose ethers (e.g., methylcellulose, hydroxyethylcellulose (HEC), methyl hydroxyethylcellulose (MHEC), ethyl hydroxyethylcellulose (EHEC), methyl ethyl hydroxyethylcellulose (MEHEC)), starch, or dextrins. Synthetic acrylate type of thickeners may as well be comprised, such as acrylate copolymers and alkyl acrylates homo or copolymers also known as associative thickeners.

The concentration of the thickening polymer is very much dependent on the type of the thickening polymer and the targeted consistency (viscosity) of the compositions. Typically, the thickening polymers are comprised in the compositions at a concentration in the range of 0.1 to 3%, preferably 0.25 to 2% by weight, calculated to the total of the composition.

In an embodiment of the present invention, the aqueous compositions advantageously comprise one or more surfactants. Suitable surfactants are selected from the classes of anionic, non-ionic, amphoteric and cationic ones.

Suitable anionic surfactants are, in principle, known from the cleansing compositions. These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates. Preferred anionic surfactants are alkyl sulphate surfactants especially lauryl sulphate and its salts.

Further suitable surfactants are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or poly-condensates of ethyleneoxide and propyleneoxide, as they are on the market, for example, under the trade name "Pluronics^{R}", as well as fatty alcohol ethoxylates, C₁₀-C₂₂-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Suitable amphoteric surfactants are various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also been proven suitable.

Suitable cationic surfactants are according to the general structure 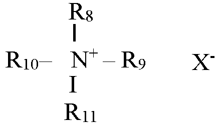 wherein R₈ is a saturated or unsaturated, branched or linear alkyl chain with 8-22 C atoms or

R₁₂ CO NH (CH₂)ₙ

where R₁₂ is saturated or unsaturated, branched or linear alkyl chain with 7-21 C atoms and n has typical value of 1 - 4 or

R₁₃ CO O (CH₂)ₙ

where R₁₃ is saturated or unsaturated, branched or linear alkyl chain with 7-21 C atoms and n has typical value of 1 - 4, and
R₉ is H or unsaturated or saturated, branched or linear alkyl chain with 1 - 22 C atoms or

   R₁₂ CO NH (CH₂)ₙ

   or

   R₁₃ CO O (CH₂)ₙ
where R₁₂, R₁₃ and n are same as above.

R₁₀ and R₁₁ are H or lower alkyl chain with 1 to 4 Carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The total concentration of one or more surfactants is in the range of 0.1 to 12.5%, preferably 0.2 to 10% and, more preferably 0.5 - 7.5% by weight, calculated to the total of the composition.

Aqueous compositions may comprise one or more fatty alcohols. Suitable fatty alcohols are the ones with the chain length of 14 to 22 C atoms which may be saturated or unsaturated, linear or branched which may, as well, be substituted. Non-limiting examples are myristyl alcohol, cetyl alcohol, stearyl alcohol, oleyl alcohol, behenyl alcohol, octyl dodecanol, cetostearyl alcohol, and their mixtures.

The total concentration of fatty alcohol is in the range from 0.5 to 15%, preferably 1 to 10% by weight, calculated to total of the composition.

Further advantageously, aqueous compositions may comprise one or more silicone compound, preferably silicone oil. Suitable and preferred ones are known with their CTFA adopted name as dimethicone and commercially available from Dow Corning under the trade name DC 200 with various viscosities.

Further silicone compounds may be comprised are aminated silicones which may be selected from amodimethicones and grafted aminated silicones. Suitable ones are available under various trade names such as DC 969, Belsil from Wacker Chemie AG and known with the CTFA adopted name Amodimethicone, and Elastomer OS from Kao Corporation known with CTFA adopted name Polysilicone-9.

Furthermore, suitably for improved hair conditioning cationic polymers known with their CTFA category name Polyquaternium may be comprised in the aqueous compositions. Typical examples of those Polyquaternium 2, Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

Preferred are Polyquaternium -2, Polyquaternium -6 and Polyquaternium 16.

The total concentration of cationic polymers may be in the range of 0.1 - 2.5%, preferably 0.25 - 2% by weight and more preferably 0.5 - 1.5% by weight, calculated to total of the composition.

Furthermore, aqueous compositions may comprise one or more organic solvent which may act as penetration enhancer and/or solubilizing agent for the compounds not readily soluble in the aqueous medium. The suitable ones are 2-phenoxyethanol, benzyl alcohol, 2-phenylethanol and 2-benzyloxyethanol. Suitable aliphatic alcohols are ethanol, isopropanol, propanol, n-butanol, isobutanol, t-butanol and 1-pentanol.

Concentration of one or more organic solvent is in the range of 0.1 to 15%, preferably 0.5 to 12.5% and more preferably 1 to 10% and, most preferably 1 to 7.5% by weight calculated to the total of the composition.

The aqueous compositions may comprise urea, at a concentration in the range of 0.1 to 20%, preferably 1 to 15% by weight calculated to the total of the compositions.

Additionally, the aqueous compositions may comprise one or more polyols. Suitable examples are glycerine, phytantriol, panthenol, ethyleneglycol, polyethyleneglycols, propylene glycols such as 1,2 propylene glycol, 1,3-propylene glycol and polypropylene glycols.

The total concentration of one or more polyol is in the range of 0.1 to 15%, preferably 0.25 to 12.5%, more preferably 0.5 to 10% and, most preferably 1 to 7.5% by weight calculated to the total of the composition.

The aqueous compositions can comprise one or more amino acids and/or their water-soluble salts. Suitable ones are glycine, histidine, citrullin, asaparagine, alanine, valine. Leucine, isoleucine, proline, tryptophan, phenylalanine, methinone, serine, tyrosine, threonine, and glutamine.

The total concentration of one or more amino acids and/or their water-soluble salts is in the range of 0.01 to 2.5%, preferably 0.1 to 2%, more preferably 0.15 to 1.5% and most preferably 0.2 to 1% by weight calculated to the total of the composition.

The aqueous compositions may comprise ingredients customarily found in such compositions such as preservative, fragrance, chelating agents, radical scavenger.

After rinsing off the reducing composition from hair and prior to application of the oxidizing composition either in waving or in straightening processes, an intermediate treatment composition is advantageously applied onto hair in order to reduce hair damage further. The intermediate composition is an aqueous composition and comprises one or more inorganic salts such as magnesium sulphate at a concentration in the range of 1 to 20% by weight, calculated to the total of the intermediate composition, and has pH in the range of 2.5 to 6, preferably 3 to 5.5 and most preferably 3 to 5.

The hair is finally treated with an oxidizing composition in order to restore the disulfide bonds. The oxidizing composition comprises at least one oxidizing agent, preferably hydrogen peroxide or sodium bromate at a concentration of 0.5 to 10% by weight calculated to total of the oxidizing composition and has pH in the range of 2 to 5, preferably 2.5 to 5 and most preferably 3 to 4.5 is applied onto hair and left on the hair 1 to 20 min and rinsed off. In cases where additional conditioning composition is required this may as well be applied after finishing the above process.

Following examples are to illustrate the invention, but not to limit it.

In each examples, the reducing composition A is a comparative composition.

### Example 1

### Reducing Composition

| | | % by weight | | | |
|---|---|---|---|---|---|
| | A | B | C | D | E |
| Ammonium thioglycolate | 12,50 | 12,50 | 12,50 | 12,50 | 12,50 |
| Monoethanolamine | 4,00 | 4,00 | 4,00 | 4,00 | 4,00 |
| Cetearyl alcohol | 4,00 | 4,00 | 4,00 | 4,00 | 4,00 |
| Ceteareth-20 | 1,50 | 1,50 | 1,50 | 1,50 | 1,50 |
| Behentrimonium chloride | 1,00 | 1,00 | 1,00 | 1,00 | 1,00 |
| Urea | 4,00 | 4,00 | 4,00 | 4,00 | 4,00 |
| EDTA | 0,20 | 0,20 | 0,20 | 0,20 | 0,20 |
| Amodimethicone | 1,50 | 1,50 | 1,50 | 1,50 | 1,50 |
| Dimethicone | 1,00 | 1,00 | 1,00 | 1,00 | 1,00 |
| Isopropyl palmiate | 0,80 | 0,80 | 0,80 | 0,80 | 0,80 |
| 1,3-butylene glycol | 1,00 | 1,00 | 1,00 | 1,00 | 1,00 |
| Ectoin | - | 0,10 | 0,50 | 1,00 | - |
| Hydroxyectoin | - | - | - | - | 0,10 |
| Water | | | q.s. to 100 | | |
| pH | 8,50 | 8,50 | 8,50 | 8,50 | 8,50 |

### Oxidizing Composition

| | % by weight |
|---|---|
| Hydrogen peroxide | 2 |
| Propylene glycol | 2,1 |
| Polyqauternium-6 | 0,4 |
| Coco betaine | 2,1 |
| Sodium cocoamphoacetate | 0,3 |
| EDTA | 0,01 |
| Phosphoric acid | 0,1 |
| Water | q.s. to 100 |
| pH | 3.0 |

The hair streaks, weighing approximately 2 g and having a length of 30 cm (Indian Frizz Hair, bought from International Hair Importers & Products Inc., NY) were straightened with the above compositions in the following way.

The hair was shampooed with a commercially available shampoo, under the brand Goldwell and towel dried, and 2 g of the above composition was applied onto hair (hair to composition ratio was 1:1 by weight) and distributed homogeneously by massaging and left on the hair for 20 min. The composition was then rinsed off from hair. The hair was dried with a hair drier and treated with a flat iron, having a surface temperature of 180°C, by passing through the hair streak 6 times, for a total of 30 sec. The oxidizing composition was then applied to the hair and processed for 10 min, and the composition was rinsed off and the hair was dried with a hair drier. It was observed that all hair streaks were straightened satisfactorily. For each composition three hair streaks were used. Tensile measurements were carried out using single fiber tensile tester, Mtt675 & Mtt680 with incorporated high frequency laser FDAS675 for cross-section measurement (DiaStron limited, UK) at 20°C, 100%RH. For each hair streak, 1 measurement was carried out with the use of 30 single fibers per streak.

In addition to the above treated streaks, a streak treated with flat iron only, as described above, was also subjected to tensile measurements.

Additionally, the durability of the straightening effect was examined after 10 times washing the hair streaks with a shampoo composition commercially available under the brand Goldwell Dual Senses for permanent shaping treated hair. The durability was calculated from the decrease in length and increase of width of the hair after 10 hair washing cycles.

The following results were obtained.

| **Composition** | **2% index** | **Durability** |
|---|---|---|
| Untreated | 43,50 | - |
| Iron treated | 40,56 | 0,00 |
| A | 28,58 | 59,8 |
| B | 29,16 | 66,9 |
| C | 28.59 | 75,8 |
| D | 29,11 | 63,5 |
| E | 28,76 | 70,5 |

After each wash cycle, the width and the length of the hair were measured, and these values used to calculate the efficacy of the treatment. The durability, in percentage, was calculated by comparing the ratio of width of the hair to the length of the hair after each wash, to the initial width and length ratio and maximal width and length ratio. Maximum width and length were measured directly after straightening (after ironing) and initial width and length were measured after 1x washing.

From the above results it is beyond any doubt that addition of Ectoin and hydroxyectoin improves hair properties in terms of elasticity expressed as 2% index and, in addition, straightening is durable in the presence of Ectoin and hydroxyectoin.

### Example 2

### Reducing Composition

| | | % by weight | | | |
|---|---|---|---|---|---|
| | A | B | C | D | E |
| Ammonium thioglycolate | 10,50 | 10,50 | 10,50 | 10,50 | 10,50 |
| Monoethanolamine | 4,30 | 4,30 | 4,30 | 4,30 | 4,30 |
| Ammonia 25% | 6,00 | 6,00 | 6,00 | 6,00 | 6,00 |
| Ammonium bicarbonate | 5,00 | 5,00 | 5,00 | 5,00 | 5,00 |
| Cetrimonium chloride | 0,70 | 0,70 | 0,70 | 0,70 | 0,70 |
| Urea | 0,25 | 0,25 | 0,25 | 0,25 | 0,25 |
| Oleic acid | 0,04 | 0,04 | 0,04 | 0,04 | 0,04 |
| Polyquaternium-16 | 0,40 | 0,40 | 0,40 | 0,40 | 0,40 |
| Dimethicone | 0,50 | 0,50 | 0,50 | 0,50 | 0,50 |
| 1,2-propylene glycol | 1,30 | 1,30 | 1,30 | 1,30 | 1,30 |
| POE(20) Oleyl ether | 0,70 | 0,70 | 0,70 | 0,70 | 0,70 |
| Ectoin | - | 0,10 | 0,50 | 1,00 | - |
| Hydroxyectoin | - | - | - | - | 0,10 |
| Water | | | q.s. to 100 | | |
| pH | 9,50 | 9,50 | 9,50 | 9,50 | 9,50 |

### Oxidizing Composition

| | % by weight |
|---|---|
| Hydrogen peroxide | 4,5 |
| Propylene glycol | 0,1 |
| Polyquaternium-6 | 0,8 |
| Coco betaine | 2,1 |
| PEG-5 Cocamide | 0,3 |
| Sodium cocoamphoacetate | 0,3 |
| Amodimethicone | 0,6 |
| VP/VA Copolymer | 0,2 |
| EDTA | 0,01 |
| Phosphoric acid | 0,1 |
| Water | q.s. to 100 |
| pH | 3.0 |

The hair streaks, weighing approximately 2 g and having a length of 28 cm (Brown Caucasian hair, bought from Fischbach + Miller GmbH & Co. KG, Germany), were permanently shaped (curled) with the above compositions in the following way.

The hair was shampooed with a commercially available shampoo, under the brand Goldwell, and towel dried. The streak was wound on to a rod with a diameter of 1.5 cm and 4 g of the above composition was applied onto hair. The hair was then left for 20 mins at ambient temperature. The composition was rinsed off from hair and the hair was towel dried. 4 g of the oxidizing lotion was applied onto the hair and left on the hair for 10 min at ambient temperature. Afterwards the hair was rinsed off and removed from the rod and the hair was shampooed once with a commercially available shampoo, under the brand Goldwell Dual Senses, and the hair was air-dried.

The obtained curls were defined with the parameter curvature index, which is the ratio of the curl width over curl depth. Curl width is the breadth of the curl and the curl depth is the length of the curve, in cm.

Durability of the curls was determined after 10 times washing the hair streaks with a commercially available shampoo composition, under the brand Goldwell Dual Senses, for permanent shaping-treated hair. The durability was expressed as the change in the curvature index.

The following results were obtained.

| | Curvature Index | |
|---|---|---|
| Composition | Inital | After 10 times washing |
| A | 0,16 | 0,14 |
| B | 0,20 | 0,17 |
| C | 0,21 | 0,18 |
| D | 0,20 | 0,17 |
| E | 0,22 | 0,17 |

From the above results the Ectoin and Hydroxyectoin improve the curvature index and also the durability of the curls.

### Example 3

### Reducing Composition

| | | % by weight | | |
|---|---|---|---|---|
| | A | B | C | D |
| Ammonium thioglycolate | 10,50 | 10,50 | 10,50 | 10,50 |
| Monoethanolamine | 4,30 | 4,30 | 4,30 | 4,30 |
| Ammonia 25% | 6,00 | 6,00 | 6,00 | 6,00 |
| Ammonium bicarbonate | 5,00 | 5,00 | 5,00 | 5,00 |
| Ectoin | - | 0,10 | 0,50 | 1,00 |
| Water | | q.s. to 100 | | |
| pH | 9,10 | 9,14 | 9,15 | 9,12 |

### Oxidizing Composition

| | % by weight |
|---|---|
| Hydrogen peroxide | 4,5 |
| Water | q.s. to 100 |
| pH | 3.0 |

The hair streaks were permanently shaped, straightened and curled, and the durability of the straightening and the curl efficiency were measured as described under Examples 1 and 2. The tensile measurements as expressed 2% index was also measured as described under Example 1.

The following results were obtained.

| | Straightening (%) | | Curling (%) | |
|---|---|---|---|---|
| Composition | 1x wash | 10x wash | 1x wash | 10x wash |
| A | 84,2 | 60,2 | 0,26 | 0,19 |
| B | 92,1 | 79,8 | 0,29 | 0,26 |
| C | 90,2 | 81,9 | 0,28 | 0,26 |
| D | 96,8 | 83,7 | 0,30 | 0,27 |

| | 2% Index | |
|---|---|---|
| Composition | Straightening | Curling |
| A | 26,12 | 27,44 |
| B | 30,58 | 29,26 |
| C | 30,76 | 27,83 |
| D | 34,26 | 27,76 |

From the above results, it is beyond any doubt that Ectoin positively effects straightening and curling of human hair and hair damage is also considerably reduced as presented by tensile measurements (2% index).

## Claims

1. An aqueous composition for the permanent shaping of human hair **characterized in that** it comprises one or more reducing agents at a total concentration in the range of 2 to 20% by weight, calculated to the total of the composition, and ectoin and/or hydroxyectoin and having a pH in the range of 7.5 to 10.5.

2. The composition according to claim 1 **characterized in that** the reducing agent is selected from thioglycolic acid, thiolactic acid and their salts, cystein and its hydrochloride salt, acetylcystein, glycerylthioglycolate and thioglycolic acid esters.

3. The composition according to claims 1 and/or 2 **characterized in that** it has a pH in the range of 8.0 to 10.5.

4. The composition according to any of the preceding claims **characterized in that** it comprises one or more reducing agent at a total concentration in the range of 2.5 to 17% by weight, calculated to the total of the composition.

5. The composition according to any of the preceding claims **characterized in that** it comprises ectoin and/or hydroxyectoin at a concentration in the range of 0.001 to 15%, preferably 0.01 to 12.5%, more preferably 0.02 to 10%, and most preferably 0.1 to 5% by weight calculated to the total of the composition.

6. The composition according to any of the preceding claims **characterized in that** it comprises at least one alkalizing agent.

7. The composition according to claim 6 **characterized in that** at least one alkalizing agent is selected form ammonia and or its salts, ammonium carbamate, ammonium(bi)carbonate, monoethanolamine and triethanolamine.

8. The composition according to any of the preceding claims **characterized in that** it comprises at least one cationic polymer, preferably selected from Polyquaternium 2, Polyquaternium 4, Polyquaternium 5, Polyquaternium 6, Polyquaternium 7, Polyquaternium 10, Polyquaternium-11, Polyquaternium 16, Polyquaternium 22, Polyquaternium 28, and Polyquaternium 39, Polyquaternium-87 and quaternized products of graft polymers from organopolysiloxanes and polyethyl oxazolines.

9. The composition according to any of the preceding claims **characterized in that** it comprises at least one surfactant selected from anionic, nonionic, cationic, and amphoteric ones, preferably at a concentration of 0.05 to 10% by weight, calculated to total composition.

10. Composition according to any of the preceding claims **characterized in that** it comprises at least one organic solvent, preferably at a concentration in the range of 0.1 to 10% by weight, calculated to total composition.

11. A method of permanent waving human hair **characterized in that**
- the hair is washed and/or shampooed and towel dried,
- a composition according to claims 1 to 10 is applied onto hair and left on the hair for 1 to 45 min, preferably 1 to 30 min,
- the hair is rinsed off
- the hair is applied an aqueous oxidizing composition comprising at least one oxidizing agent, preferably hydrogen peroxide or sodium bromate at a concentration of 0.5 to 10% by weight calculated to total of oxidizing composition, and having a pH in the range of 2 to 5, and left on the hair 1 to 20 min,
- the hair is rinsed off and dried,
wherein the hair is put on curlers before or during or after application of the composition according to claims 1 to 10 and the curlers are removed from hair during processing the oxidizing composition or after rinsing off the oxidizing composition.

12. The method according to claim 11 **characterized in that** after rinsing off the reducing agent from hair and prior to application of the oxidizing composition, an intermediate treatment composition comprising one or more inorganic salts and optionally comprising ectoin and/or hydroxyectoin and having a pH in the range of 2.5 to 6 is applied onto hair and left on the hair for 1 to 10 min and without rinsing off and after removal of the excess amount with towel, an oxidizing composition is applied.

13. A method for straightening human hair **characterized in that**
- the hair is washed and/or shampooed and towel dried,
- a composition according to claims 1 to 10 is applied onto hair and left on the hair for 1 to 45 min, preferably 1 to 30 min,
- the hair is rinsed off,
- the hair is dried with a hair drier,
- the dry hair is physically straightened with a hot iron having a surface temperature in the range of 130 to 210°C,
- the hair is applied an oxidizing composition comprising at least one oxidizing agent, preferably hydrogen peroxide or sodium bromate at a concentration of 0.5 to 10% by weight calculated to total of oxidizing composition, and having a pH in the range of 2 to 5, and left on the hair 1 to 20 min, and
- the hair is rinsed off and dried.

14. The method according to claim 13 **characterized in that** prior to application of the oxidizing composition, the hair is applied an intermediate treatment composition comprising one or more inorganic salts and optionally comprising ectoin and/or hydroxyectoin and having a pH in the range of 2.5 to 6 and left on the hair for 1 to 10 min and without rinsing off and after removal of the excess amount with towel, an oxidizing composition is applied.

15. Kit for permanent shaping human hair **characterized in that** it comprises a first product comprising a composition according to claims 1 10, a second product comprising a composition comprising at least one oxidizing agent and optionally a third product comprising a composition comprising at least one ectoin and/or hydroxyectoin.

## Patentansprüche

1. Wässrige Zusammensetzung zur dauerhaften Formgebung von menschlichem Haar, **dadurch gekennzeichnet, dass** sie ein oder mehrere Reduktionsmittel in einer Gesamtkonzentration im Bereich von 2 bis 20 Gew.-%, berechnet auf die Gesamtmenge der Zusammensetzung, und Ectoin und/oder Hydroxyectoin enthält und einen pH-Wert im Bereich von 7.5 bis 10.5 aufweist.

2. Die Zusammensetzung gemäß Anspruch 1, **dadurch gekennzeichnet, dass** das Reduktionsmittel ausgewählt ist aus Thioglykolsäure, Thiolactinsäure und deren Salzen, Cystein und dessen Hydrochloridsalz, Acetylcystein, Glycerylthioglycolat und Thioglykolsäureestern.

3. Die Zusammensetzung gemäß Anspruch 1 und/oder 2, **dadurch gekennzeichnet, dass** sie einen pH-Wert im Bereich von 8.0 bis 10.5 aufweist.

4. Die Zusammensetzung gemäß einem der vorstehenden Ansprüche, **dadurch gekennzeichnet, dass** sie ein oder mehrere Reduktionsmittel in einer Gesamtkonzentration im Bereich von 2.5 bis 17 Gew.-%, berechnet auf die Gesamtmenge der Zusammensetzung, umfasst.

5. Die Zusammensetzung gemäß einem der vorstehenden Ansprüche, **dadurch gekennzeichnet, dass** sie Ectoin und/oder Hydroxyectoin in einer Konzentration im Bereich von 0.001 bis 15 %, vorzugsweise 0.01 bis 12.5 %, noch bevorzugter 0.02 bis 10 % und am meisten bevorzugt 0.1 bis 5 Gew.-%, berechnet auf die Gesamtmenge der Zusammensetzung, umfasst.

6. Die Zusammensetzung gemäß einem der vorstehenden Ansprüche, **dadurch gekennzeichnet, dass** sie mindestens ein Alkalisierungsmittel umfasst.

7. Die Zusammensetzung gemäß Anspruch 6, **dadurch gekennzeichnet, dass** mindestens ein Alkalisierungsmittel aus Ammoniak und/oder dessen Salzen, Ammoniumcarbamat, Ammonium(bi)carbonat, Monoethanolamin und Triethanolamin ausgewählt ist.

8. Die Zusammensetzung gemäß einem der vorstehenden Ansprüche, **dadurch gekennzeichnet, dass** sie mindestens ein kationisches Polymer umfasst, vorzugsweise ausgewählt aus Polyquaternium 2, Polyquaternium 4, Polyquaternium 5, Polyquaternium 6, Polyquaternium 7, Polyquaternium 10, Polyquaternium-11, Polyquaternium 16, Polyquaternium 22, Polyquaternium 28 und Polyquaternium 39, Polyquaternium-87 und quaternisierten Produkten von Pfropfpolymeren aus Organopolysiloxanen und Polyethyl oxazolinen ausgewählt ist.

9. Die Zusammensetzung gemäß einem der vorstehenden Ansprüche, **dadurch gekennzeichnet, dass** sie mindestens ein Tensid umfasst, das aus anionischen, nichtionischen, kationischen und amphoteren Tensiden ausgewählt ist, vorzugsweise in einer Konzentration von 0.05 bis 10 Gew.-%, berechnet auf die Gesamtzusammensetzung.

10. Die Zusammensetzung gemäß einem der vorstehenden Ansprüche, **dadurch gekennzeichnet, dass** sie mindestens ein organisches Lösungsmittel umfasst, vorzugsweise in einer Konzentration im Bereich von 0.1 bis 10 Gew.-%, berechnet auf die Gesamtzusammensetzung.

11. Verfahren zum dauerhaften Wellen von menschlichem Haar, **dadurch gekennzeichnet, dass**
- Das Haar wird gewaschen und/oder mit Shampoo gewaschen und mit einem Handtuch getrocknet.
- Eine Zusammensetzung gemäß den Ansprüchen 1 bis 10 wird auf das Haar aufgetragen und 1 bis 45 Minuten, vorzugsweise 1 bis 30 Minuten, auf dem Haar belassen.
- Das Haar wird ausgespült.
- Das Haar wird mit einer wässrigen Oxidationszusammensetzung behandelt, die mindestens ein Oxidationsmittel, vorzugsweise Wasserstoffperoxid oder Natriumbromat, in einer Konzentration von 0.5 bis 10 Gew.-%, berechnet auf die Gesamtmenge der Oxidationszusammensetzung, enthält und einen pH-Wert im Bereich von 2 bis 5 aufweist, und 1 bis 20 Minuten auf dem Haar belassen wird.
- Das Haar wird ausgespült und getrocknet.
wobei das Haar vor oder während oder nach dem Auftragen der Zusammensetzung gemäß den Ansprüchen 1 bis 10 auf Lockenwickler aufgebracht wird und die Lockenwickler während der Einwirkzeit der Oxidationszusammensetzung oder nach dem Ausspülen der Oxidationszusammensetzung aus dem Haar entfernt werden.

12. Das Verfahren gemäß Anspruch 11, **dadurch gekennzeichnet, dass** nach dem Abspülen des Reduktionsmittels aus dem Haar und vor dem Auftragen der Oxidationszusammensetzung eine Zwischenbehandlungszusammensetzung, die ein oder mehrere anorganische Salze und gegebenenfalls Ectoin und/oder Hydroxyectoin enthält und einen pH-Wert im Bereich von 2.5 bis 6 aufweist, auf das Haar aufgetragen und 1 bis 10 Minuten lang auf dem Haar belassen wird, ohne abgespült zu werden, und nach Entfernung der überschüssigen Menge mit einem Handtuch eine Oxidationszusammensetzung aufgetragen wird.

13. Verfahren zum Glätten von menschlichem Haar, **dadurch gekennzeichnet, dass**
- Das Haar wird gewaschen und/oder mit Shampoo gewaschen und mit einem Handtuch getrocknet.
- Eine Zusammensetzung gemäß den Ansprüchen 1 bis 10 wird auf das Haar aufgetragen und 1 bis 45 Minuten, vorzugsweise 1 bis 30 Minuten, auf dem Haar belassen.
- das Haar wird ausgespült,
- Das Haar wird mit einem Föhn getrocknet.
- Das trockene Haar wird mit einem heißen Glätteisen mit einer Oberflächentemperatur im Bereich von 130 bis 210 °C physikalisch geglättet.
- Das Haar wird mit einer Oxidationszusammensetzung behandelt, die mindestens ein Oxidationsmittel, vorzugsweise Wasserstoffperoxid oder Natriumbromat, in einer Konzentration von 0.5 bis 10 Gew.-%, berechnet auf die Gesamtmenge der Oxidationszusammensetzung, enthält und einen pH-Wert im Bereich von 2 bis 5 aufweist, und 1 bis 20 Minuten auf dem Haar belassen wird, und
- Das Haar wird ausgespült und getrocknet.

14. Das Verfahren gemäß Anspruch 13, **dadurch gekennzeichnet, dass** vor dem Auftragen der oxidierenden Zusammensetzung auf das Haar eine Zwischenbehandlungszusammensetzung aufgetragen wird, die ein oder mehrere anorganische Salze und gegebenenfalls Ectoin und/oder Hydroxyectoin enthält und einen pH-Wert im Bereich von 2.5 bis 6 aufweist, und die 1 bis 10 Minuten auf dem Haar belassen wird, ohne sie auszuspülen, und nach dem Entfernen der überschüssigen Menge mit einem Handtuch eine Oxidationszusammensetzung aufgetragen wird.

15. Kit zur dauerhaften Formgebung von menschlichem Haar, **dadurch gekennzeichnet, dass** es ein erstes Produkt mit einer Zusammensetzung gemäß den Ansprüchen 1 bis 10, ein zweites Produkt mit einer Zusammensetzung, die mindestens ein Oxidationsmittel enthält, und gegebenenfalls ein drittes Produkt mit einer Zusammensetzung, die mindestens ein Ectoin und/oder Hydroxyectoin enthält, umfasst.

## Revendications

1. Composition aqueuse pour la mise en forme permanente des cheveux humains, **caractérisée en ce qu'**elle comprend un ou plusieurs agents réducteurs à une concentration totale comprise entre 2 et 20 % en poids, calculée par rapport au total de la composition, et de l'ectoïne et/ou de l'hydroxyectoïne, et ayant un pH compris entre 7.5 et 10.5.

2. La composition selon la revendication 1, **caractérisée en ce que** l'agent réducteur est choisi parmi l'acide thioglycolique, l'acide thiolactique et leurs sels, la cystéine et son sel chlorhydrate, l'acétylcystéine, le glycérylhexylthioglycolate et les esters d'acide thioglycolique.

3. La composition selon les revendications 1 et/ou 2, **caractérisée en ce qu'**elle a un pH compris entre 8.0 et 10.5.

4. La composition selon l'une quelconque des revendications précédentes, **caractérisée en ce qu'**elle comprend un ou plusieurs agents réducteurs à une concentration totale comprise entre 2.5 et 17 % en poids, calculée par rapport au total de la composition.

5. La composition selon l'une quelconque des revendications précédentes, **caractérisée en ce qu'**elle comprend de l'ectoïne et/ou de l'hydroxyectoïne à une concentration comprise entre 0.001 et 15 %, de préférence entre 0.01 et 12.5 %, plus préférablement entre 0.02 et 10 %, et de manière encore plus préférable entre 0.1 et 5 % en poids par rapport au total de la composition.

6. La composition selon l'une quelconque des revendications précédentes, **caractérisée en ce qu'**elle comprend au moins un agent alcalinisant.

7. La composition selon la revendication 6, **caractérisée en ce qu'**au moins un agent alcalinisant est choisi parmi l'ammoniac et/ou ses sels, le carbamate d'ammonium, le (bi)carbonate d'ammonium, la monoéthanolamine et la triéthanolamine.

8. La composition selon l'une quelconque des revendications précédentes, **caractérisée en ce qu'**elle comprend au moins un polymère cationique, de préférence choisi parmi le polyquaternium 2, le polyquaternium 4, le polyquaternium 5, le polyquaternium 6, le polyquaternium 7, le polyquaternium 10, le polyquaternium-11, polyquaternium 16, polyquaternium 22, polyquaternium 28 et polyquaternium 39, polyquaternium-87 et des produits quaternisés de polymères greffés à partir d'organopolysiloxanes et de polyéthyl oxazolines.

9. La composition selon l'une quelconque des revendications précédentes, **caractérisée en ce qu'**elle comprend au moins un tensioactif choisi parmi les tensioactifs anioniques, non ioniques, cationiques et amphotères, de préférence à une concentration de 0.05 à 10 % en poids, calculée par rapport à la composition totale.

10. Composition selon l'une quelconque des revendications précédentes, **caractérisée en ce qu'**elle comprend au moins un solvant organique, de préférence à une concentration comprise entre 0.1 et 10 % en poids, calculée par rapport à la composition totale.

11. Procédé pour la permanente des cheveux humains, **caractérisé en ce que**
- les cheveux sont lavés et/ou shampooinés et séchés à la serviette,
- une composition selon les revendications 1 à 10 est appliquée sur les cheveux et laissée sur les cheveux pendant 1 à 45 minutes, de préférence 1 à 30 minutes,
- les cheveux sont rincés
- on applique sur les cheveux une composition oxydante aqueuse comprenant au moins un agent oxydant, de préférence du peroxyde d'hydrogène ou du bromate de sodium à une concentration de 0.5 à 10 % en poids calculée par rapport à la composition oxydante totale, et ayant un pH compris entre 2 et 5, et on laisse agir sur les cheveux pendant 1 à 20 minutes,
- les cheveux sont rincés et séchés,
dans lequel les cheveux sont mis sur des bigoudis avant, pendant ou après l'application de la composition selon les revendications 1 à 10, et les bigoudis sont retirés des cheveux pendant le traitement avec la composition oxydante ou après le rinçage de la composition oxydante.

12. Procédé selon la revendication 11, **caractérisé en ce qu'**après avoir rincé l'agent réducteur des cheveux et avant d'appliquer la composition oxydante, une composition de traitement intermédiaire comprenant un ou plusieurs sels inorganiques et comprenant éventuellement de l'ectoïne et/ou de l'hydroxyectoïne et ayant un pH compris entre 2.5 et 6 est appliquée sur les cheveux et laissée sur les cheveux pendant 1 à 10 minutes sans être rincée, et après élimination de l'excès à l'aide d'une serviette, une composition oxydante est appliquée.

13. Procédé pour lisser les cheveux humains, **caractérisé en ce que**
- les cheveux sont lavés et/ou shampooinés et séchés à la serviette,
- une composition selon les revendications 1 à 10 est appliquée sur les cheveux et laissée sur les cheveux pendant 1 à 45 minutes, de préférence 1 à 30 minutes,
- les cheveux sont rincés,
- les cheveux sont séchés à l'aide d'un sèche-cheveux,
- les cheveux secs sont physiquement lissés à l'aide d'un fer chaud dont la température de surface est comprise entre 130 et 210 °C,
- on applique sur les cheveux une composition oxydante comprenant au moins un agent oxydant, de préférence du peroxyde d'hydrogène ou du bromate de sodium à une concentration de 0.5 à 10 % en poids calculée par rapport à la composition oxydante totale, et ayant un pH compris entre 2 et 5, et on laisse agir sur les cheveux pendant 1 à 20 minutes, et
- les cheveux sont rincés et séchés.

14. Procédé selon la revendication 13, **caractérisé en ce que**, avant l'application de la composition oxydante, on applique sur les cheveux une composition de traitement intermédiaire comprenant un ou plusieurs sels inorganiques et comprenant éventuellement de l'ectoïne et/ou de l'hydroxyectoïne, ayant un pH compris entre 2.5 et 6, que l'on laisse agir sur les cheveux pendant 1 à 10 minutes sans rinçage, et après avoir éliminé l'excédent à l'aide d'une serviette, une composition oxydante est appliquée.

15. Kit pour la mise en forme permanente des cheveux humains, **caractérisé en ce qu'**il comprend un premier produit comprenant une composition selon les revendications 1 à 10, un deuxième produit comprenant une composition comprenant au moins un agent oxydant et, éventuellement, un troisième produit comprenant une composition comprenant au moins une ectoïne et/ou une hydroxyectoïne.
